# DEMANDE DE BREVET EUROPEEN

(11) **EP 0 930 074 A1**
(43) Date de publication de la demande: **21.07.1999**
(21) Numéro de dépôt: 98403081.7
(22) Date de dépôt: 08.12.1998
(51) Int. Cl.: A61K 41/00, A61K 7/00

(54) **Dispositif comprenant une composition chromophore à appliquer sur la peau**

(30) Priorité: 16.12.1997 FR 9715953
(71) Demandeur: Galderma Research & Development, S.N.C., 06560 Valbonne (FR)
(72) Inventeur: Sumian, Chryslain, 180 Chemin des Combes, 06600 Antibes (FR); Pitre, Franck, 06600 Antibes (FR); Mordon, Serge, 59491 Villeneuve d'Ascq (FR)
(74) Mandataire: Leszczynski, André

(57) **Abrégé**

L'invention est relative à un dispositif permettant l'application d'une composition chromophore sur la peau, comprenant une composition chromophore contenant au moins un agent chromophore.

La composition chromophore se présente sous la forme d'une couche (2) d'épaisseur constante (e), liée par une face à un support (3), au moins avant l'application sur la peau, le ou les agents chromophores étant répartis de façon hommogène dans ladite composition et la nature de cette dernière étant choisie de manière à obtenir localement la transformation de l'énergie lumineuse d'un rayonnement laser en chaleur et/ou la modification de la structure chimique du ou des agents chromophores.

## Description

La présente invention concerne le domaine général du traitement cosmétique ou médical de la peau au moyen d'un rayonnement laser.

L'invention concerne notamment la production de chaleur à la surface de la peau au moyen d'une composition comprenant au moins un agent chromophore, appelée dans la suite composition chromophore, soumise à un rayonnement laser.

Le ou les agents chromophores absorbent l'énergie lumineuse du rayonnement laser pour la transformer en chaleur et/ou modifient leur structure chimigue sous l'effet du rayonnement laser.

Des essais réalisés en irradiant une composition chromophore avec un laser ont montré que l'on pouvait augmenter localement la température de la peau à plus de 100°C afin d'obtenir une volatilisation tissulaire, associée ou non à une coagulation du derme.

Lors de ces essais, la composition chromophore a été pulvérisée sur la peau au moyen d'un aérographe, ce qui rend très difficile l'obtention d'un dépôt régulier.

La présente invention a pour objet d'améliorer l'efficacité d'un traitement cosmétique ou médical au moyen d'une composition chromophore soumise à un rayonnement laser.

Elle y parvient grâce à un dispositif comprenant une composition chromophore et permettant l'application de cette composition chromophore sur la peau, ce dispositif étant caractérisé par le fait que la composition chromophore se présente sous la forme d'une couche d'épaisseur constante, liée par une face à un support, au moins avant l'application sur la peau, le ou les agents chromophores étant répartis de façon homogène dans ladite composition et la nature de cette dernière étant choisie de manière à obtenir localement, sous l'effet du rayonnement laser, la transformation de l'énergie lumineuse du rayonnement laser en chaleur et/ou la modification de la structure chimique du ou des agents chromophores.

L'invention permet de déposer facilement à la surface de la peau une épaisseur constante de composition chromophore.

Or, l'épaisseur de composition chromophore présente à la surface de la peau détermine dans quelle mesure le rayonnement laser est absorbé.

Le fait dans l'invention d'avoir une épaisseur constante et connue à l'avance de composition chromophore a pour avantage que l'absorbance de celle-ci est parfaitement définie, donc également la quantité de chaleur qui sera produite lors de l'irradiation par le rayonnement laser.

Ainsi, les effets du traitement par le rayonnement laser sont plus facilement déterminables et en outre le traitement est plus aisément reproductible.

Un avantage supplémentaire de l'invention réside dans le fait que la composition chromophore n'étant plus pulvérisée, elle ne risque pas de se disperser dans l'atmosphère et de se déposer ailleurs que sur la région de peau à traiter.

Dans une réalisation particulière, le dispositif comporte une ou plusieurs substances à l'état inactif en l'absence d'irradiation par le laser et aptes à se transformer en et/ou libérer sous l'effet de l'irradiation par le laser des substances ayant un effet sur la peau.

L'invention a encore peur objet un procédé pour la fabrication d'un dispositif permettant l'application d'une composition chromophore sur la peau, ce procédé étant caractérisé par le fait qu'il comporte l'étape consistant à réaliser une couche d'épaisseur constante d'une composition chromophore, la nature de cette composition étant choisie de manière à obtenir localement, sous l'effet du rayonnement laser, la transformation de l'énergie lumineuse en chaleur et/ou la modification de la structure chimique du ou des agents chromophores.

De préférence, le procédé comporte l'étape consistant à lier au moins provisoirement une couche d'épaisseur prédéterminée d'une composition chromophore à un support.

L'invention a encore pour objet un procédé pour transformer à la surface de la peau l'énergie lumineuse d'un rayonnement laser en chaleur au moyen d'une composition chromophore et/ou modifier à la surface de la peau la structure chimique d'un ou plusieurs agents chromophores contenus dans une composition chromophore, ce procédé étant caractérisé par le fait qu'il comprend les étapes consistant à :
- appliquer sur la peau une couche d'une composition chromophore, présentant une épaisseur constante et étant liée à un support au moins avant l'application sur la peau, le ou les agents chromophores étant répartis de façon homogène dans ladite composition,
- enlever éventuellement ledit support si celui-ci n'est pas transparent à la longueur d'onde du laser utilisé,
- irradier au moyen d'un laser ladite composition chromophore, l'irradiation par le laser permettant d'obtenir un dégagement de chaleur au sein de ladite couche de composition chromophore et/ou la modification de la structure chimique du ou des agents chromophores, la composition chromophore présentant à la longueur d'onde d'émission du laser une absorbance telle que l'énergie lumineuse transmise dans la peau à travers ladite couche de composition chromophore n'engendre aucun dommage tissulaire ou cellulaire irréversible non désiré.

L'invention a encore pour objet l'utilisation d'au moins un agent chromophore dans la fabrication d'une composition destinée à être appliquée sur la surface de la peau puis à être soumise à un rayonnement laser, cette composition se présentant sous la forme d'une couche ayant une épaisseur constante, l'irradiation par le laser de ladite composition permettant d'obtenir localement, au niveau de ladite composition chromophore, la transformation de l'énergie lumineuse du rayonnement laser en chaleur et/ou la modification de la structure chimique du ou des agents chromophores.

L'invention a encore pour objet un procédé de traitement cosmétique, notamment pour diminuer les rides et les ridules, caractérisé en ce qu'il comporte l'étape consistant à :
- appliquer sur la surface de la peau une composition chromophore se présentant sous la forme d'une couche ayant une épaisseur constante, la nature de la composition étant choisie de manière à obtenir localement, sous l'effet du rayonnement laser, la transformation de l'énergie lumineuse en chaleur et/ou la modification de la structure chimique du ou des agents chromophores.

Avant l'application sur la peau, la couche de composition chromophore peut être découpée au format de la région à traiter.

D'autres caractéristiques et avantages de la présente invention apparaîtront à la lecture de la description détaillée qui va suivre, d'un exemple de mise en oeuvre non limitatif de l'invention, et à l'examen du dessin annexé sur lequel :
- la figure 1 est une section transversale d'un dispositif pour l'application d'une composition chromophore sur la peau, conforme à un exemple de réalisation de l'invention,
- la figure 2 illustre le découpage du dispositif au format souhaité,
- les figures 3 à 6 illustrent l'application sur la peau et l'irradiation par un rayonnement laser.

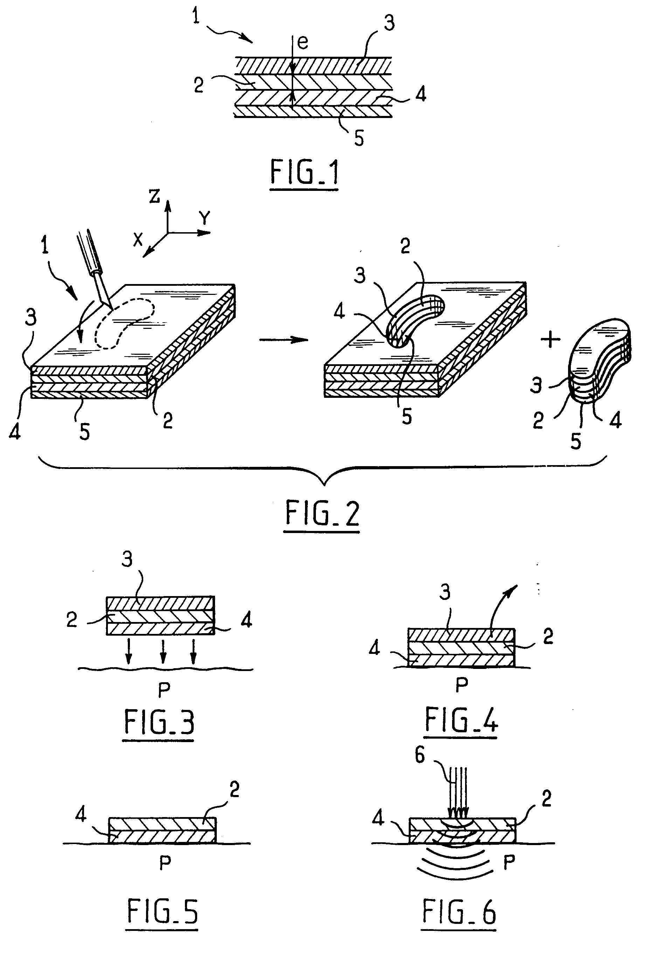

On a représenté sur les figures 1 et 2 un dispositif 1 pour l'application d'une composition chromophore sur la peau, conforme à un exemple de réalisation de l'invention, se présentant sous la forme d'une structure ou film multicouche dont l'épaisseur totale est par exemple supérieure à 100 µm, et de préférence inférieure à 3mm.

Les épaisseurs des différentes couches représentées sur les figures ont été exagérées dans un but de clarté du dessin.

Le dispositif 1 comporte une couche 2 d'une composition chromophore d'épaisseur prédéterminée, liée par une face à un support 3 et par la face opposée à une couche adhésive 4, elle-même revêtue d'un organe de protection 5.

Sur les figures, le dispositif 1 s'étend sensiblement parallèlement à un plan défini par les axes X et Y d'un repère orthogonal XYZ.

Dans ce repère XYZ, l'épaisseur e, mesurée selon l'axe Z, de la couche 2 de composition chromophore est constante quelle que soit la position selon les axes X et Y.

La composition chromophore comprend au moins un agent chromophore réparti de façon homogène au moins dans tout plan parallèle au plan XY et de préférence également selon l'axe Z dans une matrice solide, de préférence une matrice hypoallergénique et de préférence encore une matrice qui ne diffuse pas dans la peau.

Parmi les agents chromophores exogènes utilisables, on peut citer des composés minéraux tels que par exemple le noir de carbone, le graphite, l'oxyde de fer noir ou rouge ou des composés organiques tels que par exemple la mélanine, le vert d'indocyanine, des phtalocyanines et leurs complexes métalliques et plus généralement tout composé minéral ou organique absorbant suffisamment la lumière à la longueur du rayonnement laser utilisé.

Comme matrice, on peut utiliser par exemple un polymère acrylique mélangé à un dispersant destiné à homogénéiser la répartition du ou des agents chromophores au sein de la matrice.

Le ou les agents chromophores peuvent être selon leur nature et la nature de la matrice, dispersés à l'état de particules solides ou solubilisés.

La composition chromophore reste, à l'issu de la fabrication du dispositif 1, dans un état qui lui permet de conserver l'épaisseur qui lui a été donnée.

Par ailleurs, la répartition du ou des agents chromophores au sein de la matrice reste figée une fois le dispositif 1 fabriqué.

Selon la concentration en agents chromophores, l'absorbance de la couche 2 de composition chromophore est plus ou moins élevée et la quantité de chaleur produite plus ou moins grande.

La concentration en agents chromophores est choisie de manière à obtenir l'effet recherché.

Le support 3 permet de manipuler facilement le dispositif 1 avant l'application sur la peau.

De préférence, ce support 3 présente une épaisseur constante et il est suffisamment flexible pour se déformer si nécessaire pour épouser le relief de la peau lors de l'application.

Dans le mode de réalisation décrit, le support 3 est constitué par un film plastique transparent permettant de contrôler visuellement le bon collage de la couche 2 de composition chromophore sur la peau.

En variante, le support 3 peut être constitué par un tissu, tissé, ou non tissé, ou par une feuille de papier par exemple.

La couche de composition chromophore peut être autoadhésive, par exemple grâce à l'utilisation d'une matrice autoadhésive, auquel cas elle assure elle-même son maintien sur la peau P ou l'adhérence à la peau peut être obtenue, comme dans l'exemple décrit, au moyen d'une couche adhésive 4.

Cette couche adhésive 4 est de préférence liée de façon indissociable avec la couche 2 de composition chromophore et elle est constituée par une colle biocompatible, de préférence non hydrophile, afin d'éviter la modification du pouvoir collant par l'hydratation naturelle de la peau.

La couche adhésive 4 présente dans l'exemple décrit une épaisseur constante.

Les caractéristiques de résistance à la chaleur et de conduction thermique de la colle sont choisies de manière à permettre un bon transfert vers la peau de la chaleur générée au sein de la couche de composition chromophore lors de l'irradiation par le laser.

De préférence, la couche adhésive 4 est autocollante, c'est-à-dire qu'elle ne nécessite pas l'emploi d'un composé chimique extérieur pour activer son pouvoir collant.

On peut utiliser par exemple pour réaliser la couche adhésive 4 un adhésif de type SILICONES ou du type CAOUTCHOUCS SYNTHETIQUES tel que du polyisobutylène.

L'organe de protection 5 est constitué par un film relativement rigide, de préférence d'épaisseur constante, destiné à assurer la rigidité du dispositif pendant le conditionnement, la manipulation et le stockage et de faciliter par ailleurs son découpage au format souhaité si nécessaire.

L'organe de protection 5 est dans l'exemple décrit plus rigide que le support 3 et peut être constitué par une feuille de carton ou par une feuille de plastique.

Le dispositif 1 est découpable dans le sens de son épaisseur au moyen d'un outil tranchant, comme illustré par la figure 2, pour être conformé si nécessaire au contour de la région du corps à traiter.

La couche 2 de composition chromophore et le support 3 peuvent être liés par des interactions de type électrostatique ou par l'intermédiaire d'une couche adhésive.

L'adhérence entre la couche 2 de composition chromophore et le support 3 est moins forte que l'adhérence entre la couche adhésive 4 et la peau P mais plus forte que l'adhérence entre la couche adhésive 4 et l'organe de protection 5.

Si la couche 2 de composition chromophore est elle-même adhésive et fixée sur la peau sans couche adhésive 4 supplémentaire, alors l'adhérence entre le support 3 et la couche 2 de composition chromophore est inférieure à l'adhérence entre la peau et cette dernière.

Le dispositif 1 s'utilise de la manière suivante.

On découpe si nécessaire, à l'aide d'un outil tranchant, la structure multicouche au format souhaité, comme illustré sur la figure 2.

Une fois ce découpage réalisé, l'organe de protection 5 est enlevé et la couche adhésive 4 est appliquée sur la peau P.

Ensuite le support 3 est enlevé, laissant apparaître la couche 2 de composition chromophore, assujettie à la peau P par le biais de la couche adhésive 4.

La souplesse de l'ensemble constitué par la couche 2 de composition chromophore et la couche adhésive 4 lui permet de suivre le relief cutané.

Au moyen d'un laser, on irradie la couche 2 de composition chromophore, ce qui produit un dégagement de chaleur au sein de celle-ci, lequel se propage par conduction dans la peau P.

On peut ainsi augmenter localement la température de la peau à plus de 100°C par exemple afin d'obtenir une volatilisation associée ou non avec une coagulation du derme.

De préférence on utilise un laser dont l'irradiance est inférieure à 10⁸W/cm2.

Le ou les agents chromophores utilisés sont de préférence choisis pour faire en sorte que la fraction du rayonnement laser qui traverse la couche 2 de composition chromophore n'engendre aucun dommage tissulaire ou cellulaire irréversible non désiré dans la peau P.

Selon le traitement à effectuer, on peut utiliser une couche 2 de composition chromophore présentant une épaisseur plus ou moins grande, de manière à obtenir le dégagement de chaleur recherché, par exemple une profondeur plus ou moins grande de volatilisation et/ou de coagulation tissulaire.

Le dispositif 1 selon l'invention peut s'utiliser pour éliminer un caractère inesthétique de la peau tel que des rides, des ridules, des verrues, des cicatrices atrophiques et/ou hypertrophiques.

Il peut également être utilisé pour traiter, éventuellement en complément d'un traitement médicamenteux, des pathologies cutanées telles que le rhinophyma, une hyperkératose, des hyperproliférations cutanées, une plaque de psoriasis, un cancer cutané, une kératose actinique ou encore des kéloïdes.

Il peut encore être utilisé dans le but d'augmenter la pénétration d'actifs cosmétiques ou pharmaceutiques, plus particulièrement dermatologiques.

Le ou les agents chromophores utilisés sont adaptés à la longueur d'onde du rayonnement laser utilisé et à l'effet recherché.

On peut utiliser des lasers émettant à des longueurs d'ondes égales par exemple à 585 nm, 694 nm, 532 nm, 10,6 µm, 2,94 µm, 2,12 µm ou encore 1,06 µm, le ou les agents chromophores étant choisis de manière à absorber suffisamment l'énergie lumineuse du rayonnement laser à la longueur d'onde considérée.

On peut obtenir une couche 2 de composition chromophore d'épaisseur constante lors de la fabrication du dispositif 1 par calandrage ou extrusion par exemple.

La couche 2 de composition chromophore peut être conformée à l'épaisseur souhaitée avant d'être assujettie au support 3 ou en variante la composition chromophore peut être déposée sur le support 3 puis calandrée avec ce dernier à l'épaisseur souhaitée.

La couche adhésive 4 peut être déposée par enduction sur la couche 2 de composition chromophore.

Le dispositif 1 est avantageusement proposé au praticien dans un conditionnement stérile.

Chaque conditionnement correspond à une épaisseur donnée de composition chromophore et l'on peut proposer au praticien une gamme de dispositifs 1 ayant des épaisseurs différentes, pour lui permettre de choisir l'épaisseur qui convient le mieux au résultat recherché.

Bien entendu, l'invention n'est pas limitée à l'exemple de réalisation qui vient d'être décrit.

On peut notamment, dans le cas où le support 3 est entièrement transparent et inerte à la longueur d'onde du rayonnement laser utilisé, laisser ce dernier en place sur la couche 2 de composition chromophore lors de l'irradiation par le rayonnement laser.

De préférence, le support 3 est alors poreux pour laisser passer l'oxygène pouvant être nécessaire au traitement.

Dans une variante non illustrée, la couche de composition chromophore peut être appliquée directement sur la peau, sans interposition d'une couche adhésive appartenant au dispositif.

Toujours dans une variante non illustrée, le support peut être microporeux et lié à la couche de composition chromophore grâce à une couche adhésive qui est dissoute partiellement ou totalement par un solvant imbibant le support, afin de permettre le retrait de ce dernier avant l'irradiation par un laser.

Le dispositif peut comporter en outre une ou plusieurs substances actives exerçant une activité sur la peau avant ou après l'irradiation par le laser.

Cette ou ces substances actives peuvent exercer leur effet lors de l'application sur la peau et/ou changer d'état sous l'effet du rayonnement laser et pénétrer alors dans la peau.

Cette ou ces substances actives peuvent être contenues dans la composition chromophore et/ou la couche adhésive qui permet l'adhésion de la composition chromophore à la peau, lorsque la composition chromophore n'est pas adhérente en elle-même.

Il peut s'agir par exemple d'un anesthésiant ou d'un cicatrisant.

L'invention permet aussi de mettre en oeuvre une photothérapie dynamique consistant en l'utilisation d'un produit pharmaceutique en association avec un laser, par exemple de l'HPD (hémato-porphyrine dérivé) qui, présente à l'état inactif dans le dispositif, se transforme sous l'effet du rayonnement laser en une substance active ayant des vertus curatives et/ou cosmétiques.

Le rayonnement laser peut encore produire un dégagement de chaleur au sein de la composition chromophore pour faciliter la pénétration dans la peau du ou des substances actives contenues dans le dispositif.

## Revendications

1. Dispositif permettant l'application d'une composition chromophore sur la peau, comprenant une composition chromophore contenant au moins un agent chromophore, caractérisé par le fait que la composition chromophore se présente sous la forme d'une couche (2) d'épaisseur constante (e), liée par une face à un support (3), au moins avant l'application sur la peau, le ou les agents chromophores étant répartis de façon homogène dans ladite composition et la nature de cette dernière étant choisie de manière à obtenir localement la transformation de l'énergie lumineuse d'un rayonnement laser en chaleur et/ou la modification de la structure chimique du ou des agents chromophores.

2. Dispositif selon la revendication 1, caractérisé par le fait que ladite couche de composition chromophore (2) est liée de façon amovible audit support (3).

3. Dispositif selon l'une quelconque des revendications précédentes, caractérisé par le fait que ladite composition chromophore est autoadhésive.

4. Dispositif selon la revendication 1 ou 2, caractérisé par le fait que ladite couche (2) de composition chromophore est liée, par sa face opposée audit support, à une couche adhésive (4).

5. Dispositif selon la revendication 4, caractérisé par le fait que ladite couche adhésive (4) et la couche (2) de composition chromophore sont liées de façon indissociable.

6. Dispositif selon l'une quelconque des revendications 4 et 5, caractérisé par le fait que les caractéristiques d'adhérence de ladite couche adhésive (4) sont choisies de telle sorte que ladite couche adhésive (4) adhère plus fortement à la peau (P) que le support (3) n'adhère à la couche (2) de composition chromophore.

7. Dispositif selon l'une quelconque des revendications 4 à 6, caractérisé par le fait qu'il comporte un organe de protection amovible (5) revêtissant ladite couche adhésive (4) et par le fait que les caractéristiques d'adhérence de ladite couche adhésive (4) sont choisies de telle sorte que ladite couche adhésive (4) adhère plus fortement à la couche (2) de composition chromophore qu'audit organe de protection (5).

8. Dispositif selon la revendication 7, caractérisé par le fait que ledit organe de protection (5) est plus rigide que le support (3).

9. Dispositif selon l'une quelconque des revendications 1 à 8, caractérisé par le fait que ledit support (3) est transparent à la longueur d'onde du laser utilisé.

10. Dispositif selon l'une quelconque des revendications 1 à 9, caractérisé par le fait que ledit support (3) est suffisamment souple pour pouvoir épouser le relief cutané.

11. Dispositif selon l'une quelconque des revendications 1 à 10, caractérisé par le fait qu'il comporte une ou plusieurs substances aptes à diffuser dans la peau sous l'action de la chaleur dégagée lors de l'irradiation par le laser de la composition chromophore.

12. Dispositif selon l'une quelconque des revendications précédentes, caractérisé par le fait qu'il comporte une ou plusieurs substances pharmaceutiques actives independamment de l'action du laser, telles qu'un anesthésiant ou un cicatrisant.

13. Dispositif selon l'une quelconque des revendications précédentes, caractérisé par le fait qu'il comporte une ou plusieurs substances à l'état inactif en l'absence d'irradiation par le laser et aptes à se transformer en et/ou libérer sous l'effet de l'irradiation par le laser des substances actives ayant un effet sur la peau.

14. Procédé pour la fabrication d'un dispositif permettant l'application d'une composition chromophore comprenant au moins un agent chromophore sur la peau, caractérisé par le fait qu'il comporte l'étape consistant à réaliser une couche (2) d'épaisseur constante d'une composition chromophore, le ou les agents chromophores étant répartis de façon homogène dans ladite composition, la nature de cette dernière étant choisie de manière à obtenir localement, la transformation de l'énergie lumineuse d'un rayonnement laser en chaleur et/ou la modification de la structure chimique du ou des agents chromophores.

15. Procédé selon la revendication 14, caractérisé par le fait qu'il comporte l'étape consistant à lier au moins provisoirement une couche d'épaisseur prédéterminée d'une composition chromophore à un support (3).

16. Procédé pour transformer à la surface de la peau l'énergie lumineuse d'un rayonnement laser en chaleur au moyen d'une composition chromophore comprenant au moins un agent chromophore, et/ou modifier à la surface de la peau la structure chimique d'un ou plusieurs agents chromophores contenus dans une composition chromophore, caractérisé par le fait qu'il comprend les étapes consistant à :
- appliquer sur la peau une couche d'une composition chromophore, présentant une épaisseur constante et liée à un support au moins avant l'application sur la peau, le ou les agents chromophores étant répartis de façon homogène dans ladite composition,
- enlever éventuellement ledit support si celui-ci n'est pas transparent à la longueur d'onde du laser utilisé et,
- irradier au moyen d'un laser ladite composition chromophore, l'irradiation par le laser permettant d'obtenir la transformation de l'énergie lumineuse du rayonnement laser en chaleur au sein de ladite couche de composition chromophore, et/ou la modification de la structure chimique du ou des agents chromophores, ladite composition chromophore présentant à la longueur d'onde d'émission du laser une absorbance telle que l'énergie lumineuse transmise dans la peau a travers ladite couche de composition chromophore n'engendre aucun dommage tissulaire ou cellulaire irréversible non désiré.

17. Procédé selon la revendication 16, caractérisé par le fait que ledit support est poreux et par le fait que son enlèvement s'effectue au moyen d'un solvant.

18. Utilisation d'au moins un agent chromophore dans la fabrication d'une composition destinée à être appliquée sur la surface de la peau puis à être soumise à un rayonnement laser, cette composition chromophore se présentant sous la forme d'une couche ayant une épaisseur constante, le ou les agents chromophores étant répartis de façon homogène dans ladite composition, l'irradiation produite par le laser permettant d'obtenir localement, au niveau de ladite composition, la transformation de l'énergie lumineuse du rayonnement laser en chaleur, et/ou la modification de la structure chimique du ou des agents chromophores.

19. Procédé de traitement cosmétique, notamment pour diminuer les rides et les ridules, caractérisé en ce qu'il comporte l'étape consistant à :
- appliquer sur la surface de la peau une composition comprenant au moins un agent chromophore, cette composition se présentant sous la forme d'une couche (2) ayant une épaisseur constante, le ou les agents chromophores étant répartis de façon homogène dans ladite composition, la nature de cette dernière étant choisie de manière à obtenir localement, sous l'effet du rayonnement laser, la transformation de l'énergie lumineuse en chaleur et/ou la modification de la structure chimique du ou des agents chromophores.

20. Procédé selon la revendication 19, caractérisé par le fait qu'avant l'application sur la peau, la couche (2) de composition chromophore est découpée au format de la région à traiter.
